# EUROPÄISCHE PATENTANMELDUNG

(11) **EP 0 542 216 A1**
(43) Veröffentlichungstag der Anmeldung: **19.05.1993**
(21) Anmeldenummer: 92119266.2
(22) Anmeldetag: 11.11.1992
(51) Int. Cl.: C07C 327/32, A61K 49/02, C07C 323/60, C07B 59/00, C07F 13/00

(54) **Diaminomercapto (thio)ether als Komplexbildner für in der Nuklearmedizin einsetzbare Nuklide, Verfahren zur Herstellung der Komplexe, sowie Markierungskits, enthaltend diese Komplexe**

(30) Priorität: 13.11.1991 DE 4137267
(71) Anmelder: HOECHST AKTIENGESELLSCHAFT, D-65926 Frankfurt am Main (DE)
(72) Erfinder: Brandau, Wolfgang, Dr., W-4400 Münster (DE); Fischer, Sabine, W-4410 Warendorf (DE)

(57) **Zusammenfassung**

Es werden Diaminomercapto(thio)ether als neue Komplexbildner für Nuklide beschrieben, die in der Nuklearmedizin Anwendung finden.

## Beschreibung

Die Erfindung betrifft Diaminomercapto(thio)ether als neue Komplexbildner für in der Nuklearmedizin einsetzbare Nuklide.

Die erfolgreiche Behandlung von Erkrankungen des Menschen setzt die genaue Diagnose des jeweils vorliegenden Krankheitsprozesses voraus. Dabei spielen häufig bildgebende Verfahren eine herausragende Rolle, da damit ohne operativen Eingriff die sonst nicht sichtbaren Organe dargestellt werden können.

Die zur Zeit üblichen Methoden werden in zwei Gruppen eingeteilt. Auf der einen Seite stehen die Verfahren, mit denen raumfordernde Prozesse abgebildet werden. Dazu gehören insbesondere das Röntgen, der Ultraschall und die Computer-Tomographie (CT). Die auf der Resonanz von Atomkernen im Magnetfeld beruhende NMR-Diagnostik kann hier ebenfalls eingeordnet werden. Auf der anderen Seite stehen die nuklearmedizinischen Verfahren, die nach Injektion eines mit einem radioaktiven Nuklid markierten Präparates die vom Nuklid ausgehende Strahlung mit einer speziellen Kamera (γ-Kamera) registrieren und über eine mathematische Auswertung in ein Bild umsetzen. Da in diesem Falle Präparate appliziert werden, die zwangsläufig in irgendeiner Form am Metabolismus des betreffenden Organismusses teilnehmen, spiegelt das erhaltene Bild den physiologischen Zustand wider, der bei vielen Krankheiten sehr viel früher ein abnormales Verhalten zeigt, als dies durch eine Veränderung der morphologischen Struktur sichtbar wird.

Diese Verfahren zeichnen sich daher durch die Möglichkeit einer frühzeitigen Diagnosestellung und einer bildlichen Beschreibung des Funktionszustandes der betreffenden Organe aus. Da in der NMR-Diagnostik mit Kontrastmitteln auf Basis bestimmter Nuklide (bevorzugt Gadolinium) gearbeitet wird, ist unter gewissen Umständen auch diese Methode in der Lage, gleichartige Aussagen zu liefern.

Die in der Nuklearmedizin oder NMR-Diagnostik einsetzbaren Nuklide müssen eng eingrenzbare Kriterien erfüllen. In Abhängigkeit von der jeweiligen Fragestellung werden u. a. die folgenden Nuklide verwendet: ^{99m}Tc, ⁶⁸Ga, ⁶⁷Ga, ¹¹¹In, ^{113m}In, ¹⁸⁶Re, ¹⁸⁸Re, ⁶⁷Cu, ⁶⁴Cu, ⁶²Cu, ¹⁵³Gd oder andere stabile Gadoliniumisotope.

Eine herausragende Bedeutung in der Nuklearmedizin besitzt dabei das Technetium-99m. Aufgrund seiner günstigen Halbwertszeit (6,02 Stunden), optimalen γ-Energie (140 keV) und allgemeinen Verfügbarkeit (⁹⁹Mo/^{99m}Tc-Generator) hat es größte Verbreitung gefunden.

Die Applikation des aus dem Generator zur Verfügung stehenden Technetium-99m, das chemisch als Pertechnetat vorliegt, genügt jedoch nicht allen Anforderungen der nuklearmedizinischen Diagnostik, da damit nur wenige Organe des Menschen untersucht werden können. Durch Komplexierung des Technetium-99m mit einem geeigneten Liganden werden jedoch in der Wirkung vollkommen unterschiedliche Verbindungen erhalten. Da vorwiegend nur einzelne Organe oder begrenzte Organsysteme zu untersuchen sind, um diese in einen Zusammenhang mit der vermuteten Krankheit stellen zu können, müssen diese Komplexe eine hohe Organspezifität aufweisen. In den letzten Jahrzehnten sind eine Reihe von Komplexbildnen für radioaktive Nuklide entwickelt worden, insbesondere für Tc-99 m, die eine hohe Organspezifität aufweisen. Andererseits gibt es aber noch eine Reihe von Fragestellungen, für die noch keine optimalen Präparate gefunden worden sind. Hierzu gehört insbesondere die bildliche Darstellung des Durchblutungszustandes von Herz und Gehirn und die quantitative Erfassung der tubulären Sekretionsleistung der Nieren. Die auf diesen Gebieten erzielten Fortschritte werden von A.M. Verbruggen zusammengefaßt (Eur. J. Nucl. Med. 17, 346-364 (1990).

Darüberhinaus sind Komplexe mit Ethylencystein aus WO 90/05733, mit Mercaptoacetyltriglycin aus EP-A-0 250 013, EP-A-0 427 360, EP-A-0 173 424 und US-A-4 883 862, mit Diamiddithiolaten aus EP-A-0 200 492 und EP-A-0 135 160 sowie mit Bisamindithiolen aus WO 89/10759, EP-A-0 200 211, WO 89/10758, EP-A-0 279 417, EP-A-0 322 876, EP-A-0 344 724 und EP-A-0 163 119 bekannt.

Aus dem genannten Stand der Technik ist die Lehre zu entnehmen, daß eine Komplexierung des Zentralatoms über primäre oder sekundäre Aminofunktionen und/oder über Thiolfunktionen am besten erfolgt.

Aufgabe der Erfindung war es nun, Komplexbildner zu finden, die ebenso wie die Verbindungen des Standes der Technik in der Lage sind mit Metallen, insbesondere mit Technetium, stabile Komplexe zu bilden.

Die Lösung der Aufgabe besteht in der Bereitstellung von Komplexbildnern der allgemeinen Formel I, die chelatisierend wirken und als funktionelle Gruppen sekundäre und tertiäre Amine und Ether- und/oder Thioetherfunktionen enthalten.

Die Erfindung betrifft Verbindungen der allgemeinen Formel I 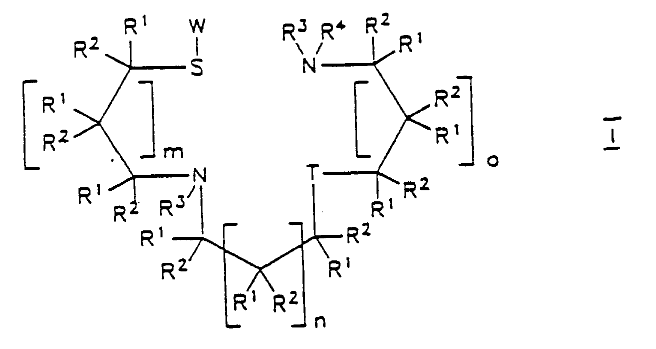
wobei:
- T: S oder O,
- R₁: zusammen mit R₂ doppelt gebundenes O oder S oder
- R₁, R₂, R₃, R₄: gleich oder verschieden sind und
H, -(CH₂)ₐ-CH₃, -COOR', -CONR'R'', OR', - SO₃R', -SO₂NR'R'', -OCOR', -CONHCH₂COOH, -SR', -NR'R'' oder -COR' bedeuten, wobei R' und R'' gleich oder verschieden sind und H, - (CH₂)_{b}CH₃, Phenyl, p-Hydroxyphenyl, N-Piperidinyl, N-Piperazinyl oder N-Morpholinyl bedeuten,
- W: H, -COCH₃, -COC₆H₅, -CH₂NHCOCH₃, -CH₂C₆H₅, - COCH₂OH, -COCH₂COOH oder eine andere geeignete Schwefelschutzgruppe, sowie
- m, n, o: unabhängig voneinander 0, 1 oder 2 und
- a, b: unabhängig voneinander 0 bis 20, vorzugsweise 0 bis 10 bedeuten.

Bevorzugt sind Verbindungen der allgemeinen Formel I, in denen
- T: S
- R₁,: zusammen mit R₂ doppelt gebundenes O oder
- R₁, R₂, R₃, R₄: gleich oder verschieden sind und
-H, -(CH₂)ₐCH₃ - COOR', -CONR'R'', -OR', - NR'R'', wobei R' und R'' gleich oder verschieden sind und H, -(CH₂)_{b}CH₃, Phenyl, p-Hydroxyphenyl bedeuten.
- W: H, -COCH₃, -COC₆H₅, -COCH₂OH, -CH₂-C₆H₅ oder - COCH₂COOH bedeuten,
sowie
- m, n, o: gleich sind und
- a, b: 0 bis 5
bedeuten.

Insbesondere bevorzugt sind Verbindungen der allgemeinen Formel I, in denen R₁ zusammen mit R₂ nur an den Positionen O bedeutet, an denen sich eine Carbonylamido-Struktur 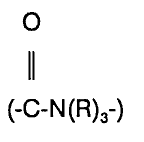
ausbildet.

Hierunter sind wiederum diejenigen Verbindungen bevorzugt, in denen die anderen Positionen R₁ und R₂ Wasserstoff, -COOR', -OR', -COR', insbesondere Wasserstoff oder -COOR', bedeuten, und R' die oben genannte Bedeutung hat.

R₃ und R₄ bedeuten hierbei vorzugsweise Wasserstoff oder -(CH₂)ₐ-CH₃, wobei a eine Zahl zwischen 1 und 5 ist.

Ganz besonders bevorzugt sind Verbindungen der allgemeinen Formel I, in denen m, n und o gleich sind und 0 oder 1, insbesondere 0 bedeutet.

Von den Schwefelschutzgruppen W sind besonders bevorzugt -COCH₃, -COC₆H₅, oder -CH₂C₆H₅.

Die erfindungsgemäßen Komplexbildner sind besonders geeignet zur Ausbildung von stabilen Komplexen mit ^{99m}Tc, ⁶⁸Ga, ¹¹¹In, ¹¹³In, ¹⁸⁶Re, ¹⁸⁸Re, ⁶⁷Cu, ¹⁵³Gd oder stabilen Gadoliniumisotopen.

Die hieraus entstehenden Komplexe sind auch Gegenstand der vorliegenden Erfindung.

Die erfindungsgemäßen Komplexe zeichnen sich dadurch aus, daß Sie durch tubuläre Sekretion über die Nieren ausgeschieden werden. Insbesondere ist auch hier der entsprechende Komplex mit Tc-99m zu nennen.

Durch Variation von Zentralatom und Substitutionsmuster des Komplexbildners nach der allgemeinen Formel I sind auch Komplexe zugänglich, die sich besonders gut zur Darstellung des Durchblutungszustandes von Herz und Gehirn eignen.

Die erfindungsgemäßen Komplexe entsprechen der folgenden allgemeinen Formel II 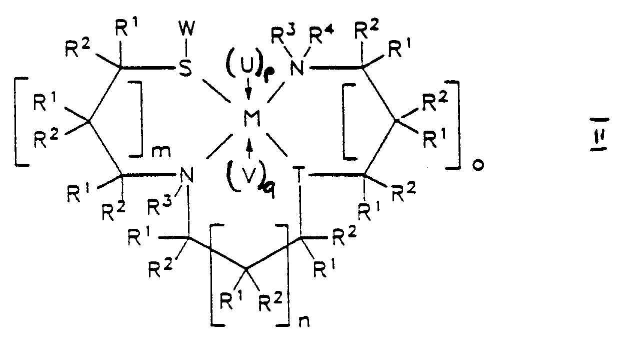
worin
- R₁, R₂, R₃, R₄, W, m, n, o: die oben angegebenen Bedeutungen haben und
- M: ^{99m}Tc, ⁶⁸Ga, ¹¹¹In, ¹¹³In, ¹⁸⁶Re, ¹⁸⁸Re, ⁶⁷Cu, ⁶⁴Cu, ⁶²Cu, ¹⁵³Gd oder ein stabiles Isotop des Gadolinium und
- U und V: doppelt gebundenes O oder S, dreifach gebundenes N, - PXY oder H₂O, CH₃CH₂OH, CH₃OH, Cl, Br oder I bedeuten, worin
- X und Y: -OR', -H, -(CH₂)_{b}CH₃, -(CH₂)ₐ-O-(CH₂)_{b}-CH₃, wobei R' die o. g. Bedeutung hat und a, b unabhängig voneinander 0-10 bedeuten und
- p und q: 0 oder 1 bedeuten.

Vorzugsweise ist U und V entweder O oder S sowie p und q jeweils 1 oder p = 1 und q = 0 oder p und q gleich 0.

Die erfindungsgemäßen Komlexbildner können frei oder mit einer Schutzgruppe für die Thiolfunktion für die Komplexierung eingesetzt werden. Die Schutzgruppe wird vor dem Markierungsvorgang abgespalten. Da in dem Kohlenstoffgerüst Chiralitätszentren eingeführt werden können, ist sowohl die Bildung von racemischen Gemischen als auch optisch reinen Isomeren möglich.

Die Synthese der Komplexbildner geht von allgemein verwendbaren Vorstufen aus, die zu den gewünschten Endprodukten umgesetzt werden können. Vorstufen dieser Art sind z. B. das Succinimidyl-S-benzoylthioglycolat oder S-Benzoylmercaptoethylthioglycolsäureamid. Das folgende Schema zeigt die Synthese des im Beispiel 1 aufgeführten Komplexbildners aus einer dieser Vorstufen. Die Synthese der Vorstufen selbst wird im folgenden beschrieben.

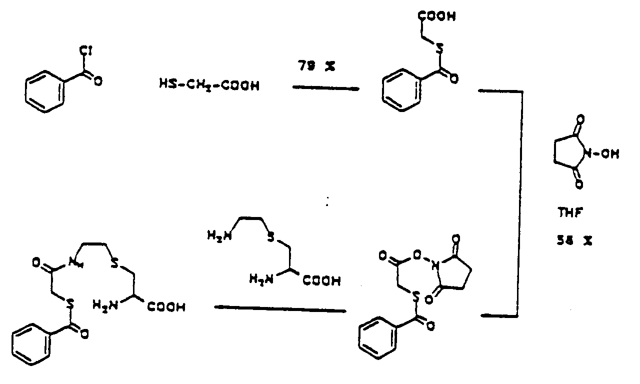

### Herstellung der S-Benzoylthioglycolsäure

In einer Mischung aus 75 ml Wasser und 75 ml Toluol werden 8.88 g (0.22 mol) Natriumhydroxidplätzchen gelöst. Zu der Lösung werden 9,2 g (0,1 mol) Mercaptoessigsäure gegeben und 9,2 ml (0,1 mol) Benzoylchlorid bei 5-15°C innerhalb von 30 Minuten zugetropft. Es wird für 30 Minuten bei 5-15°C und weitere 30 Minuten bei Raumtemperatur gerührt. Danach gibt man Wasser hinzu, trennt die Phasen und schüttelt die wässrige Phase mit Toluol aus. Die wässrige Phase wird mit konz. Salzsäure auf pH = 1-1.5 eingestellt, der Niederschlag abgesaugt und aus Ethylacetat umkristallisiert. Das Produkt wird als farblose Kristalle erhalten.
Ausbeute: 12,8 g (65 %)
Schmp.: 99°C (Lit. (J. Nucl. Med. 25, 223-229 (1984): 102-103°C)

### Herstellung des Succinimidyl-S-benzoylthioglycolat

In 70 ml trockenem Tetrahydrofuran werden 11 g (0,056 mol) S-Benzoylthioglycolsäure und 6,46 g (0,056 mol) N-Hydroxysuccinimid bei -5° bis 0°C gelöst. Danach tropft man innerhalb von 30 Minuten bei dieser Temperatur 13,75 g (0,066 mol) Dicyclohexylcarbodiimid gelöst in 15 ml Tetrahydrofuran zu. Man läßt noch zwei Stunden bei -5° bis 0°C und 18 Stunden bei Raumtemperatur rühren. Nach Zugabe von 0,2 ml Eisessig läßt man eine Stunde rühren und saugt dann den ausgefallenen Dicyclohexylharnstoff ab, kocht den Niederschlag zweimal mit Tetrahydrofuran aus und engt die Filtrate im Vakuum ein. Nach Umkristallisation aus Ethylacetat erhält man das Produkt als farblose Kristalle.
Ausbeute: 11,5 g (69 %)
Schmp.: 131-133°C

### Herstellung von S-Benzoyl-mercaptoethylthioglycolsäureamid

In 300 ml Tetrahydrofuran werden 14,65 g (0,05 mol) Succinimidyl-S-benzoylthioglycolat und 4,5 g (0,058 mol) Mercaptethylamin gelöst und 21 Stunden unter Rückfluß gekocht. Die Reaktionslösung wird eingeengt, das Produkt in Chloroform aufgenommen und mehrmals mit Wasser gewaschen. Die organische Phase wird über Natriumsulfat getrocknet und eingeengt. Das als Öl anfallende Produkt wird aus Acetonitril umkristallisiert und über eine Kieselgelsäule mit Acetonitril endgültig gereinigt.

Die Synthese der Metallkomplexe wird durch die Eigenschaften des Nuklids bestimmt. Während Technetium-99m und Rhenium-186 die Reduktion des allgemein zur Verfügung stehenden Pertechnetats-^{99m}Tc bzw. Perrhenats-¹⁸⁶Re erfordern, die mit Zinn(II) ausgeführt wird, können Metalle wie Indium, Gallium, Kupfer und Gadolinium direkt mit dem Komplexbildner umgesetzt werden, da sie bereits in Oxidationsstufen vorliegen, die eine Komplexierung ermöglichen.

Die Markierungskits können nach den üblichen Verfahren hergestellt werden. Dazu wird der Komplexbildner in einer wässrigen Lösung vorgelegt und entweder gefroren gelagert oder lyophilisiert. Die Gefriertrocknung stellt eine bevorzugte Anwendungsform dar, da sie sich durch eine besonders gute Haltbarkeit und bequeme Handhabung auszeichnet. Die Abspaltung der Schutzgruppe kann sowohl vor der Lyophilisierung als auch während des eigentlichen Markierungsvorganges stattfinden. Die Beispiele zeigen, daß es für die Komplexierung keine Rolle spielt, ob die Schutzgruppe vor Zugabe des Nuklids oder danach entfernt wird.

### Beispiel 1

### Synthese von 10-Benzoyl-8-keto-7-aza-2-amino-4, 10-dithiadekansäure

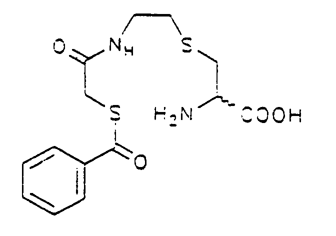

In 5 ml Wasser werden 500 mg (2.5 mmol) S-Aminoethyl-L-cystein-.hydrochlorid gelöst und mit 2,5 ml (2.5 mmol) 1 N Natriumhydroxidlösung in die freie Base überführt. Zu der Lösung werden langsam 732.5 mg (2.5 mmol) Succinimidyl-S-benzoylthioglycolat gelöst in 25 ml Tetrahydrofuran gegeben und 20 Stunden bei Raumtemperatur gerührt. Der farblose Niederschlag des Produktes wird abfiltriert und zweimal mit Wasser und Ethanol gewaschen. Nach Umkristallisation des Rohprodukts aus Methanol erhält man 523 mg (1.59 mmol, 64 %) der oben bezeichneten Säure.
Schmelzpunkt: 200 - 201°C
Massenspektrum (70 eV, 210°C): m/e = 298 (M⁺-CO₂), 281 (M⁺-CO₂-NH₃), 255 (M⁺-NH₂CH(COOH)CH₂), 207 (M⁺-NH₂CH(COOH)CH₂SCH₂) und 196 (C₆H₅COSCH₂CONH₃)
¹H-NMR (DMSO-d₆, ppm): 7.92 (Benzyl, 2H), 7.71 (Benzyl, 1H), 7.57 (Benzyl, 2H), 3.83 (CH₂, 2H), 3.29 (CH₂, 2H), 3.06 (CH₂, 2H) und 2.66 (CH₂, 2H)
HPLC (Säule RP-18,5 µm, 250 x 4.6 mm / Vorsäule: 20 x 4.6 mm); 0.05 m Phosphatpuffer pH = 7.0 : Acetonitril = 82 : 18 (v/v):Rₜ = 15.8 Minuten

### Beispiel 2

### Herstellung eines Technetium-99m Komplexes mit Abspaltung der Schutzgruppe nach Zugabe von Pertechnetat-^{99m}Tc.

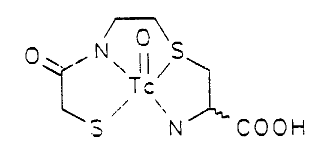

In 450 µl Wasser werden 1-2 mg der nach Beispiel 1 hergestellten Verbindung suspendiert und durch Zugabe von 150 µl 1 N Natronlauge gelöst. Zu der Lösung gibt man 10 µl einer SnCl₂-Lösung (10 mg SnCl₂ x 2 H₂O in 1 ml 0,1 N Salzsäure) und 0,1-2 ml Pertechnetat-^{99m}Tc (100-9000 MBq). Die Mischung wird für 2-30 Minuten auf 100°C erhitzt und nach dem Abkühlen auf Raumtemperatur auf einen pH-Wert von pH = 4-8 neutralisiert.

Der Technetium-99m Komplex wird mit einer radiochemischen Reinheit von > 96 % erhalten. Die Überprüfung mit HPLC belegte die Stabilität des Komplexes in organischen, wässrigen oder biologischen (Humanserumalbumin) Lösungen über 24 Stunden.
HPLC (System: Siehe Beispiel 1): Rₜ = 5.2 Minuten (Pertechnetat-^{99m}Tc: Rₜ = 4.1 Minuten); Wiederfindung: 97 % der aufgegebenen Aktivitätsmenge
DC (ITLC SG / 2-Butanon)
R_{f}-Komplex = 0, R_{f}-Pertechnetat-^{99m}Tc = 1, R_{f}kolloidales ^{99m}Tc = 0
DC (ITLC SG / 0.9 % Natriumchloridlösung)
R_{f}-Komplex = 1, R_{f}-Pertechnetat-^{99m}Tc = 1, R_{f}kolloidales ^{99m}Tc = 0

### Beispiel 3

### Herstellung eines Technetium-99m Komplexes mit Abspaltung der Schutzgruppe vor Zugabe von Pertechnetat-^{99m}Tc.

In 450 µl Wasser werden 1-2 mg der nach Beispiel 1 hergestellten Verbindung suspendiert und durch Zugabe von 150 µl 1 N Natronlauge gelöst. Anschließend wird die Lösung für 2 - 20 Minuten auf 100°C erhitzt. Nach Abkühlen auf Raumtemperatur werden 10 µl SnCl₂-Lösung (10 mg SnCl₂ x 2 H₂O in 1 ml 0,1 N Salzsäure) und 0,1 - 2 ml Pertechnetat-^{99m}Tc (100 - 9000 MBq) zugefügt. Die Lösung bleibt für 2 - 30 Minuten bei Raumtemperatur stehen und wird dann auf einen pH-Wert von pH = 4 - 8 neutralisiert.

Der Komplex wird mit einer radiochemischen Reinheit von > 95 % erhalten. Die gefundenen Ergebnisse der HPLC und DC entsprechen den Werten von Beispiel 2.

### Beispiel 4

### Herstellung des Gallium-67-Komplexes

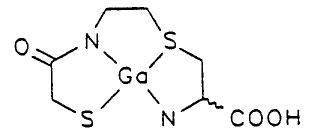

In 450 µl Wasser werden 1-2 mg der nach Beispiel 1 hergestellten Substanz gegeben und durch Zugabe von 150 µl 1 N Natronlauge gelöst. Die Lösung wird für 2 - 30 Minuten auf 100°C erhitzt. Nach Abkühlen auf Raumtemperatur wird die Lösung angesäuert (pH = 2 - 5), 0.2 - 2 ml ⁶⁷Ga-Citrat (37 - 740 MBq) hinzugefügt und für 2 - 30 Minuten bei Temperaturen zwischen 20 und 100°C stehengelassen. Der Komplex wird mit einer radiochemischen Reinheit von > 80 % erhalten und ist nach den Ergebnissen der DC mehr als 5 Stunden stabil.
DC (ITLC SG / Wasser)
R_{f}-Komplex = 0.1, R_{f}-⁶⁷Ga-Citrat = 0.0, R_{f}-⁶⁷Ga-Hydroxid = 0.0
DC (ITLC SG / 0,05 M Phosphatpuffer (pH = 7.0): Acetonitril = 82 : 18)
R_{f}-Komplex = 0.2, R_{f}-⁶⁷Ga-Citrat = 1.0, R_{f}-⁶⁷Ga-Hydroxid = 0.0
DC (Whatman Nr. 1 / Wasser)
R_{f}-Komplex = 0.8, R_{f}-⁶⁷Ga-Citrat = 1.0, R_{f}-⁶⁷Ga-Hydroxid = 0.6

### Beispiel 5

### Herstellung des Indium-111-Komplexes

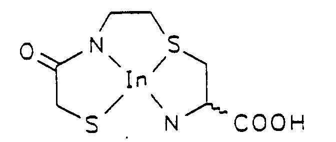

In 450 µl Wasser werden 1-2 mg der nach Beispiel 1 hergestellten Substanz gegeben und durch Zugabe von 150 µl 1 N Natronlauge gelöst. Die Lösung wird für 2 - 30 Minuten auf 100°C erhitzt. Nach Abkühlen auf Raumtemperatur wird die Lösung angesäuert (pH = 2 - 5), 0.1 - 1 ml ¹¹¹In-Chlorid (74-740 MBq) hinzugefügt und für 2-30 Minuten bei Temperaturen zwischen 20 und 100°C stehengelassen. Der Komplex wird mit einer radiochemischen Reinheit von > 70 % erhalten und ist nach den Ergebnissen der DC mehr als 4 Stunden stabil.
DC (reversed phase, RP-18 / 0,05 M Phosphaptpuffer pH = 7 : Acetonitril = 82:18)
R_{f}-Komplex = 0.1, R_{f}-¹¹¹InCl₃ = 0.2, R_{f}-¹¹¹In-Hydroxid = 0.0
DC (ITLC SG / 0,05 M Phosphaptpuffer pH = 7 : Acetonitril = 82 : 18)
R_{f}-Komplex = 1.0, R_{f}-¹¹¹InCl₃ = 0.0, R_{f}-¹¹¹In-Hydroxid = 0.0

### Beispiel 6

### Herstellung des Rhenium-186-Komplexes

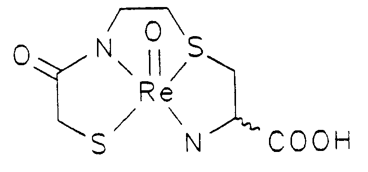

In 0,45 ml Wasser werden durch Zugabe von 0,15 ml wässriger 1N Natronlauge 1-2 mg der in Beispiel 1 hergestellten Verbindung gelöst und für 2-30 Minuten auf 100°C erwärmt, um die Schutzgruppe abzuspalten. Nach dem Abkühlen auf Raumtemperatur wird auf pH = 2-5 angesäuert, 10 µl SnCl₂ (40-60 mg SnCl₂ x 2 H₂O in 1 ml 1 N Salzsäure lösen) und 0,1-1 ml (37-740 MBq) Perrhenat-¹⁸⁶Re zugegeben. Nach Reaktion für 2-30 Minuten bei Temperaturen zwischen 20° und 100°C wird der Komplex mit einer Reinheit von > 60 % erhalten. Die Überprüfung der in vitro Stabilität zeigte, daß der Komplex über 24 Stunden stabil ist.
DG (ITLC SG/2-Butanon)
R_{f}-Komplex = 0, R_{f}-Perrhenat-¹⁸⁶Re = 1, R_{f}-kolloidales ¹⁸⁶Re = 0
DC (ITLC SG/0,9 % Natriumchloridlösung)
R_{f}-Komplex = 1, R_{f}-Perrhenat-¹⁸⁶Re = 1, R_{f}-kolloidales ¹⁸⁶Re = 0

## Patentansprüche

1. Verbindungen der allgemeinen Formel I 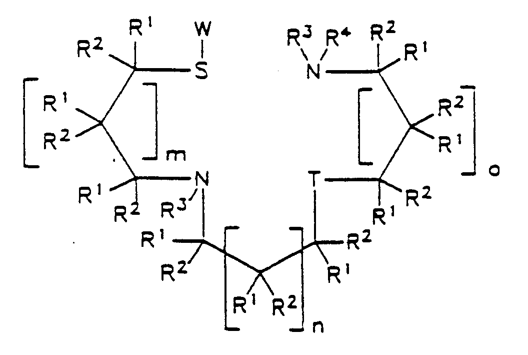 wobei:
T S oder O,
R₁ zusammen mit R₂ doppelt gebundenes O oder S oder
R₁, R₂, R₃, R₄ gleich oder verschieden sind und
H, -(CH₂)ₐ-CH₃, -COOR', -CONR'R'', OR', - SO₃R', SO₂NR'R'', -OCOR', -CONHCH₂COOH, -SR', - NR'R'' oder -COR' bedeuten, wobei
R' und R'' gleich oder verschieden sind und H, - (CH₂)_{b}CH₃, Phenyl, p-Hydroxyphenyl, N-Piperidinyl, N-Piperazinyl oder N-Morpholinyl bedeuten,
W H, -COCH₃, -COC₆H₅, -CH₂NHCOCH₃, -CH₂C₆H₅, - COCH₂OH, -COCH₂COOH oder eine andere geeignete Schwefelschutzgruppe, sowie
m, n, o unabhängig voneinander 0, 1 oder 2 und
a, b unabhängig voneinander 0 bis 20, vorzugsweise 0 bis 10 bedeuten.

2. Verbindungen nach Anspruch I, in denen
T S
R₁, zusammen mit R₂ doppelt gebundenes O oder
R₁, R₂, R₃, R₄ gleich oder verschieden sind und
-H, -(CH₂)ₐCH₃ - COOR', -CONR'R'', - OR', -NR'R'', wobei R' und R'' gleich oder verschieden sind und H, -(CH₂)_{b}CH₃, Phenyl, p-Hydroxyphenyl bedeuten.
W H, -COCH₃, -COC₆H₅, -COCH₂OH, -CH₂C₆H₅ oder - COCH₂COOH bedeuten,
sowie
m, n, o gleich sind und
a, b 0 bis 5
bedeuten.

3. Verbindungen nach den Ansprüchen 1 oder 2, in denen R₁ zusammen mit R₂ nur an den Positionen O bedeutet, an denen sich eine Carbonylamido-Struktur 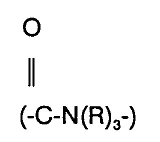 ausbildet.

4. Verbindungen nach Anspruch 3, in denen die anderen Positionen R₁ und R₂ Wasserstoff, -COOR', -OR', -COR' und R₃ und R₄ Wasserstoff oder -(CH₂)ₐ-CH₃ bedeuten, wobei a 1 bis 5 bedeutet, und R' die oben genannte Bedeutung hat.

5. Verbindungen nach den Ansprüchen 1 bis 4, in denen m, n und o gleich sind und 0 oder 1 bedeuten.

6. Komplexe der allgemeinen Formel II 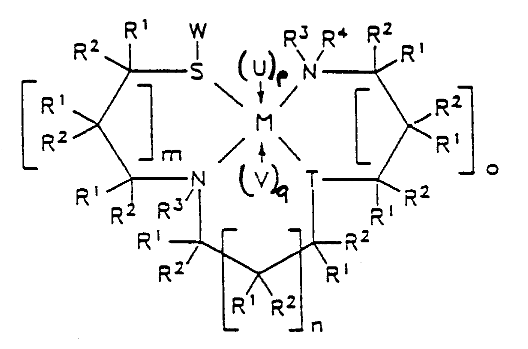 worin
R₁, R₂, R₃, R₄, W, m, n, o die Bedeutungen gemäß den Ansprüchen 1 bis 6 haben und
M ^{99m}Tc, ⁶⁸Ga, ¹¹¹In, ¹¹³In, ¹⁸⁶Re, ¹⁸⁸Re, ⁶⁷Cu, ⁶⁴Cu, ⁶²Cu, ¹⁵³Gd oder ein stabiles Isotop des Gadolinium und
U und V doppelt gebundenes O oder S, dreifach gebundenes N, - PXY oder H₂O, CH₃CH₂OH, CH₃OH, Cl, Br oder I bedeutet, worin
X und Y -OR', -H, (CH₂)_{b}CH₃, -(CH₂)ₐ-O(CH₂)_{b}-CH₃, wobei R' die o. g. Bedeutung hat, und a, b unabhängig voneinander 0-10 bedeuten.
p und q 0 oder 1 bedeuten.

7. Komplexe der allgemeinen Formel II gemäß Anspruch 6, in denen
U und V entweder O oder S und
p und q jeweils 1 oder
p = 1 und
q = O oder
p und q gleich 0 bedeuten.

8. Komplexe nach den Ansprüchen 6 und 7, in denen M ^{99m}Tc ist.

9. Verfahren zur Herstellung der Komplexe gemäß den Ansprüchen 6 bis 8, dadurch gekennzeichnet, daß eine Verbindung gemäß den Ansprüchen 1 bis 5 mit einem Metall M zusammen gebracht wird.

10. Verfahren nach Anspruch 9, dadurch gekennzeichnet, daß M Pertechnetat - ^{99m}Tc ist.

11. Verwendung der Komplexe gemäß den Ansprüchen 6 bis 8 in der medizinischen Diagnostik.

12. Markierungskit, enthaltend mindestens einen Komplex gemäß den Ansprüchen 6 bis 8.

## Patentansprüche (Patentansprüche für folgende(n) Vertragsstaat(en): ES)

1. Verfahren zur Herstellung von Verbindungen der allgemeinen Formel II 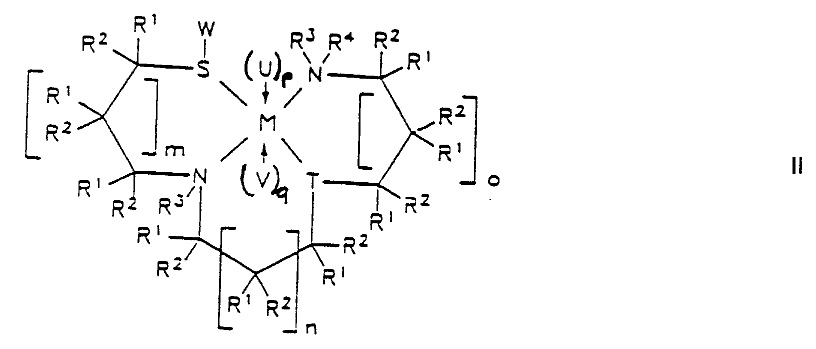 wobei:
T S oder O,
R₁ zusammen mit R₂ doppelt gebundenes O oder S oder
R₁, R₂, R₃, R₄ gleich oder verschieden sind und
H, -(CH₂)ₐ-CH₃, -COOR', -CONR'R'', OR', - SO₃R', SO₂NR'R'', -OCOR', -CONHCH₂COOH, -SR', - NR'R'' oder -COR' bedeuten, wobei
R' und R'' gleich oder verschieden sind und H, - (CH₂)_{b}CH₃, Phenyl, p-Hydroxyphenyl, N-Piperidinyl, N-Piperazinyl oder N-Morpholinyl bedeuten,
W H, -COCH₃, -COC₆H₅, -CH₂NHCOCH₃, -CH₂C₆H₅, - COCH₂OH, -COCH₂COOH oder eine andere geeignete Schwefelschutzgruppe, sowie
m, n, o unabhängig voneinander 0, 1 oder 2 und
a, b unabhängig voneinander 0 bis 20, vorzugsweise 0 bis 10 bedeutet,
U und V doppelt gebundenes O oder S, dreifach gebundenes N, - PXY oder H₂O, CH₃CH₂OH, CH₃OH, Cl, Br oder I bedeutet, worin
X und Y -OR', -H, (CH₂)_{b}CH₃, -(CH₂)ₐ-O-(CH₂)_{b}-CH₃, wobei R' die o. g. Bedeutung hat, und a, b unabhängig voneinander 0-10 bedeuten und
p und q 0 oder 1 bedeuten,
dadurch gekennzeichnet, daß man eine Verbindung der Formel I 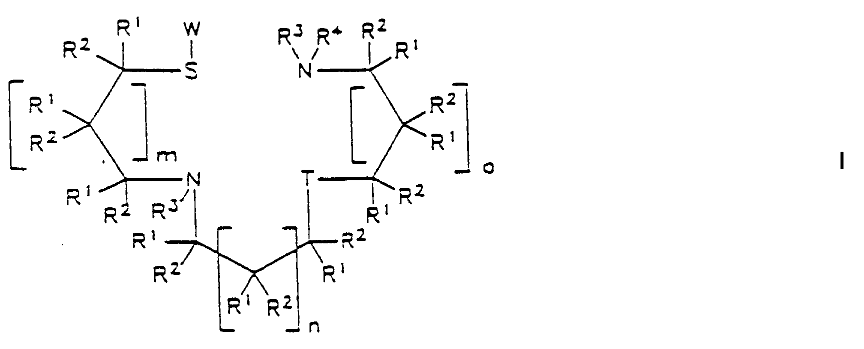 mit einem Metall M, wobei
M ^{99m}Tc, ⁶⁸Ga, ¹¹¹In, ¹¹³In, ¹⁸⁶RE, ¹⁸⁸Re, ⁶⁷Cu, ⁶⁴Cu, ⁶²Cu, ¹⁵³Gd oder ein stabiles Isotop des Gadolinium bedeutet,
umsetzt.

2. Verfahren nach Anspruch 1, dadurch gekennzeichnet, daß
T S
R₁, zusammen mit R₂ doppelt gebundenes O oder
R₁, R₂, R₃, R₄ gleich oder verschieden sind und
-H, -(CH₂)ₐCH₃ - COOR', -CONR'R'', - OR', -NR'R'', wobei R' und R'' gleich oder verschieden sind und H, -(CH₂)_{b}CH₃, Phenyl, p-Hydroxyphenyl bedeuten,
W H, -COCH₃, -COC₆H₅, -COCH₂OH, -CH₂C₆H₅ oder - COCH₂COOH bedeuten,
sowie
m, n, o gleich sind und
a, b 0 bis 5
bedeuten.

3. Verfahren nach den Ansprüchen 1 oder 2, in dem R₁ zusammen mit R₂ nur an den Positionen O bedeutet, an denen sich eine Carbonylamido- Struktur 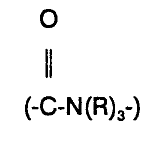 ausbildet.

4. Verfahren nach Anspruch 3, in dem die Positionen R₁ und R₂ Wasserstoff, - COOR', -OR'-COR' und R₃ und R₄ Wasserstoff oder -(CH₂)ₐ-CH₃ bedeutet, wobei a 1 bis 5 bedeutet, und R' die in Anspruch 1 genannte Bedeutung hat.

5. Verfahren nach den Ansprüchen 1 bis 4, in dem m, n und o gleich sind und 0 oder 1 bedeutet.

6. Verfahren nach den Ansprüchen 1 bis 5, in dem
U und V entweder O oder S und
p und q jeweils 1 oder
p = 1 und
q = O oder
p und q gleich 0 bedeutet.

7. Verfahren nach den Ansprüchen 1 bis 6, in dem M ^{99m}Tc ist.

8. Verfahren nach Anspruch 7, dadurch gekennzeichnet, daß M Pertechnetat - ^{99m}Tc ist.

9. Verwendung der Komplexe gemäß den Ansprüchen 1 bis 8 in der medizinischen Diagnostik.

10. Markierungskit, enthaltend mindestens einen Komplex gemäß den Ansprüchen 1 bis 8.
